# EUROPÄISCHE PATENTSCHRIFT

(11) **EP 3 080 306 B1**
(45) Veröffentlichungstag und Bekanntmachung des Hinweises auf die Patenterteilung: **31.10.2018**
(21) Anmeldenummer: 14815579.9
(22) Anmeldetag: 11.12.2014
(51) Int. Cl.: C01B 3/12, C01B 3/18, C01B 3/56, C21B 7/00, C21B 5/06, C01B 3/38, C21C 5/28, C21C 5/38, C12P 7/06

(54) **ANLAGENVERBUND ZUR STAHLERZEUGUNG UND VERFAHREN ZUM BETREIBEN DES ANLAGENVERBUNDES**
COMBINED SYSTEM FOR PRODUCING STEEL AND METHOD FOR OPERATING THE COMBINED SYSTEM
ENSEMBLE D'INSTALLATIONS PERMETTANT LA PRODUCTION D'ACIER ET PROCÉDÉ PERMETTANT DE FAIRE FONCTIONNER L'ENSEMBLE D'INSTALLATIONS

(30) Priorität: 12.12.2013 DE 102013113958
(43) Veröffentlichungstag der Anmeldung: 19.10.2016
(73) Patentinhaber: thyssenkrupp AG, 45143 Essen (DE)
(72) Erfinder: ACHATZ, REINHOLD, 45259 Essen (DE); WAGNER, Jens, 60439 Frankfurt a.M. (DE); OLES, Markus, 45525 Hattingen (DE); SCHMÖLE, Peter, 44141 Dortmund (DE); KLEINSCHMIDT, Ralph, 45472 Mülheim a.d.R. (DE); GEHRMANN, Stefan, 44137 Dortmund (DE); KOLBE, Bärbel, 58452 Witten (DE); KRÜGER, Matthias, Patrick, 44625 Herne (DE)
(74) Vertreter: thyssenkrupp Intellectual Property GmbH
(86) Internationale Anmeldenummer: PCT/EP2014/003316
(87) Internationale Veröffentlichungsnummer: WO 2015/086150

(56) Entgegenhaltungen:
- EP-A1- 2 657 215
- WO-A1-00/05421
- FR-A1- 2 420 568

## Beschreibung

Die Erfindung betrifft einen Anlagenverbund zur Stahlerzeugung sowie ein Verfahren zum Betreiben des Anlagenverbundes.

Der Anlagenverbund zur Stahlerzeugung umfasst einen Hochofen zur Roheisenerzeugung, ein Konverterstahlwerk zur Rohstahlerzeugung, ein Gasleitungssystem für Gase, die bei der Roheisenerzeugung und/oder der Rohstahlerzeugung anfallen, sowie ein Kraftwerk zur Stromerzeugung. Das Kraftwerk ist als Gasturbinenkraftwerk oder Gasturbinen- und Dampfturbinenkraftwerk ausgelegt und wird mit einem Gas betrieben, welches zumindest eine Teilmenge des bei der Roheisenerzeugung im Hochofen anfallenden Hochofengichtgases und/oder eine Teilmenge des in dem Konverterstahlwerk anfallenden Konvertergases umfasst.

Im Hochofen wird aus Eisenerzen, Zuschlägen sowie Koks und anderen Reduktionsmitteln wie Kohle, Öl, Gas, Biomassen, aufbereitetem Altkunststoffen oder sonstigem Kohlenstoff und/oder Wasserstoff enthaltenden Stoffen Roheisen gewonnen. Als Produkte der Reduktionsreaktionen entstehen zwangsläufig CO, CO₂, Wasserstoff und Wasserdampf. Ein aus dem Hochofenprozess abgezogenes Hochofengichtgas weist neben den vorgenannten Bestandteilen häufig einen hohen Gehalt an Stickstoff auf. Die Gasmenge und die Zusammensetzung des Hochofengichtgases ist abhängig von den Einsatzstoffen und der Betriebsweise und unterliegt Schwankungen. Typischerweise enthält Hochofengichtgas jedoch 35 bis 60 Vol.-% N₂, 20 bis 30 Vol.-% CO, 20 bis 30 Vol.-% CO₂ und 2 bis 15 Vol.-% H₂. Rund 30 bis 40% des bei der Roheisenerzeugung entstehenden Hochofengichtgases wird im Regelfall zum Aufheizen des Heißwindes für den Hochofenprozess in Winderhitzern eingesetzt; die verbleibende Gichtgasmenge kann in anderen Werksbereichen zu Heizzwecken oder zur Stromerzeugung genutzt werden.

Im Konverterstahlwerk, das dem Hochofenprozess nachgeschaltet ist, wird Roheisen zu Rohstahl umgewandelt. Durch Aufblasen von Sauerstoff auf flüssiges Roheisen werden störende Verunreinigungen wie Kohlenstoff, Silizium, Schwefel und Phosphor entfernt. Da die Oxidationsprozesse eine starke Wärmeentwicklung verursachen, wird häufig Schrott in Mengen bis zu 25 % bezogen auf das Roheisen als Kühlmittel zugesetzt. Ferner werden Kalk zur Schlackenbildung und Legierungsmittel zugegeben. Aus dem Stahlkonverter wird ein Konvertergas abgezogen, welches einen hohen Gehalt an CO aufweist und ferner Stickstoff, Wasserstoff und CO₂ enthält. Eine typische Konvertergaszusammensetzung weist 50 bis 70 Vol.-% CO, 10 bis 20 Vol.-% N₂, ca. 15 Vol.-% CO₂ und ca. 2 Vol.-% H₂ auf. Das Konvertergas wird entweder abgefackelt oder bei modernen Stahlwerken aufgefangen und einer energetischen Nutzung zugeführt.

Der Anlagenverbund kann optional im Verbund mit einer Kokerei betrieben werden. In diesem Fall umfasst der eingangs beschriebene Anlagenverbund zusätzlich eine Koksofenanlage, in der Kohle durch einen Verkokungsprozess in Koks umgewandelt wird. Bei der Verkokung von Kohle zu Koks fällt ein Koksofengas an, welches einen hohen Wasserstoffgehalt und beachtliche Mengen an CH₄ enthält. Typischerweise enthält Koksofengas 55 bis 70 Vol.-% H₂, 20 bis 30 Vol.-% CH₄, 5 bis 10 Vol.-% N₂ und 5 bis 10 Vol.-% CO. Zusätzlich weist das Koksofengas Anteile von CO₂, NH₃ und H₂S auf. In der Praxis wird das Koksofengas in verschiedenen Werksbereichen zu Heizzwecken und im Kraftwerksprozess zur Stromerzeugung genutzt. Darüber hinaus ist es bekannt, Koksofengas zusammen mit Hochofengichtgas oder mit Konvertergas zur Erzeugung von Synthesegasen zu verwenden. Gemäß einem aus WO 2010/136313 A1 bekannten Verfahren wird Koksofengas aufgetrennt in einen wasserstoffreichen Gasstrom und einen CH₄ und CO enthaltenen Restgasstrom, wobei der Restgasstrom dem Hochofenprozess zugeführt wird und der wasserstoffreiche Gasstrom mit Hochofengichtgas gemischt und zu einem Synthesegas weiterverarbeitet wird. Aus EP 0 200 880 A2 ist es bekannt, Konvertergas und Koksofengas zu mischen und als Synthesegas für eine Methanolsynthese zu nutzen. Aus WO 00/05421 ist es bekannt, Hochofengas zu nutzen um einen Kraftwerk, eine Chemieanlage und eine biotechnologische Anlage zu betrieben.

In einem integrierten Hüttenwerk, welches im Verbund mit einer Kokerei betrieben wird, werden etwa 40 bis 50% der als Hochofengichtgas, Konvertergas und Koksofengas anfallenden Rohgase für verfahrenstechnische Prozesse eingesetzt. Etwa 50 bis 60% der entstehenden Gase werden dem Kraftwerk zugeführt und zur Stromerzeugung genutzt. Der im Kraftwerk erzeugte Strom deckt den Strombedarf für die Roheisen- und Rohstahlerzeugung. Im Idealfall ist die Energiebilanz geschlossen, so dass abgesehen von Eisenerzen und Kohlenstoff in Form von Kohle und Koks als Energieträger kein weiterer Eintrag von Energie notwendig ist und außer Rohstahl und Schlacke kein Produkt den Anlagenverbund verlässt.

Vor diesem Hintergrund liegt der Erfindung die Aufgabe zugrunde, die Wirtschaftlichkeit des Gesamtprozesses weiter zu verbessern und einen Anlagenverbund anzugeben, mit dem es möglich ist, die Kosten für die Stahlerzeugung zu reduzieren.

Ausgehend von einem Anlagenverbund zur Stahlerzeugung mit einem Hochofen zur Roheisenerzeugung, einem Konverterstahlwerk zur Rohstahlerzeugung, einem Gasleitungssystem für Gase, die bei der Roheisenerzeugung und/oder der Rohstahlerzeugung anfallen, und einem Kraftwerk zur Stromerzeugung sind an das Gasleitungssystem erfindungsgemäß eine Chemieanlage und eine biotechnologische Anlage angeschlossen, wobei das Kraftwerk, die Chemieanlage und die biotechnologische Anlage hinsichtlich der Gasversorgung in einer Parallelschaltung angeordnet sind. Das Gasleitungssystem umfasst erfindungsgemäß eine betrieblich steuerbare Gasverteilungsvorrichtung zur Aufteilung der dem Kraftwerk, der Chemieanlage und der biotechnologischen Anlage zugeführten Gasmengenströme. Vorteilhafte Ausgestaltungen des erfindungsgemäßen Anlagenverbundes werden in den Ansprüchen 2 bis 4 beschrieben.

Gegenstand der Erfindung ist auch ein Verfahren nach Anspruch 5 zum Betreiben eines Anlagenverbundes, der einen Hochofen zur Roheisenerzeugung, ein Konverterstahlwerk, eine Chemieanlage, eine biotechnologische Anlage und ein Kraftwerk aufweist. Gemäß dem erfindungsgemäßen Verfahren wird zumindest eine Teilmenge des bei der Roheisenerzeugung im Hochofen anfallenden Hochofengichtgases und/oder eine Teilmenge des bei der Rohstahlerzeugung anfallenden Konvertergases als Nutzgas zum Betrieb des Kraftwerkes, der Chemieanlage und der biotechnologischen Anlage verwendet. Ein erster Teilstrom des Nutzgases wird der Chemieanlage zugeführt und nach einer Gasaufbereitung als Synthesegas zur Herstellung chemischer Produkte verwendet. Ein zweiter Teilstrom des Nutzgases wird im Kraftwerk zur Stromerzeugung eingesetzt. Ein dritter Teilstrom des Nutzgases wird der biotechnologischen Anlage zugeführt und für biochemische Prozesse verwendet. Der dritte Teilstrom kann mit oder ohne Gasaufbereitung für biochemische Prozesse eingesetzt werden. Im Falle einer Änderung des dem Kraftwerk zugeführten Gasstromes werden der zweite Teilstrom und der dritte Teilstrom des Nutzgases wechselweise verändert, so dass die Chemieanlage mit einer Teilstrommenge des Nutzgases betrieben werden kann, die geringeren betrieblichen Schwankungen ausgesetzt ist als der in der biotechnologischen Anlage verwertete Nutzgasteilstrom. Zweckmäßig wird der dritte Nutzgasteilstrom so geregelt, dass der in der Chemieanlage eingesetzte erste Nutzgasteilstrom mit einer Schwankungsbreite von ± 20 % konstant bleibt.

In der Chemieanlage werden chemische Produkte aus einem Synthesegas erzeugt, welche jeweils die Komponenten des Eduktes enthalten. Chemische Produkte können beispielsweise Ammoniak oder Methanol oder auch andere Kohlenwasserstoffverbindungen sein.

Mit einer biotechnologischen Anlage ist eine Anlage zur Fermentation von Synthesegas gemeint, welches CO und H₂ als Hauptbestandteile enthält. Aus diesem Synthesegas können ebenfalls Kohlenwasserstoffverbindungen, beispielsweise Ethanol, Aceton und dergleichen erzeugt werden. Der Wasserstoffanteil stammt dabei allerdings im Wesentlichen aus Wasser, das bei der Fermentation als Medium eingesetzt wird. Zur Herstellung des Synthesegases benötigt man daher ein Gas, das einen hohen Anteil an CO aufweist. Vorzugsweise wird Konvertergas oder ein Mischgas aus Konvertergas und Hochofengichtgas eingesetzt.

Der im Kraftwerk zur Stromerzeugung verwendete Nutzgasteilstrom unterliegt erheblichen betrieblichen Schwankungen. Der von dem Kraftwerk erzeugte Strom deckt einen Teil des Strombedarfes des Anlagenverbundes. Zusätzlich wird externer Strom bezogen, der vorzugsweise vollständig oder zumindest teilweise aus erneuerbarer Energie gewonnen wird und beispielweise aus Windkraftanlagen, Solaranlagen, geothermischen Kraftwerken, Wasserkraftwerken, Gezeitenkraftwerken und dergleichen stammt. Zur Erreichung eines möglichst wirtschaftlichen Betriebs des Anlagenverbundes wird der Kraftwerksbetrieb gedrosselt, wenn externer Strom in ausreichender Menge und zu günstigen Preisen zur Verfügung steht. Wenn Strom aus regenerativen Quellen nicht in ausreichendem Maße zur Verfügung steht bzw. externer Strom einen höheren Preis als im Kraftwerk erzeugbarer Strom hat, wird das Kraftwerk hochgefahren und der überwiegende Teil des Nutzgases im Kraftwerksprozess zur Stromerzeugung eingesetzt. Der Anteil des Nutzgases, der als Synthesegas zur Herstellung chemischer Produkte verwendet werden kann, unterliegt daher im Ergebnis erheblichen betrieblichen Schwankungen, die durch den Kraftwerksbetrieb vorgegeben werden.

Die dynamische Regelung einer Chemieanlage bei Lastwechseln ist technisch aufwendig. Das Problem, dass eine im Verbund mit einem Kraftwerk betriebene Chemieanlage auf Lastwechsel des Kraftwerkes nicht ausreichend flexibel reagieren kann, wird erfindungsgemäß dadurch gelöst, dass bei einem Lastwechsel des Kraftwerks zunächst nur die Leistung der Biotechnologieanlage angepasst wird und dass der für die biotechnologische Anlage bestimmte Nutzgasteilstrom und der im Kraftwerk eingesetzte Nutzgasteilstrom wechselweise verändert werden, so dass die Chemieanlage mit einer Teilstrommenge des Nutzgases betrieben werden kann, die wesentlich geringeren betrieblichen Schwankungen ausgesetzt ist als der in der biotechnologischen Anlage verwertete Nutzgasteilstrom. Die erfindungsgemäße Lehre nutzt dabei aus, dass eine Biotechnologieanlage im Vergleich zu einer Chemieanlage hinsichtlich Lastwechsel wesentlich flexibler ist.

Gemäß einer bevorzugten Ausführung der Erfindung umfasst der Anlagenverbund zusätzlich eine Koksofenanlage. Wenn die Roheisenerzeugung und die Rohstahlerzeugung im Verbund mit einer Kokerei betrieben wird, kann eine Teilmenge des bei der Roheisenerzeugung anfallenden Hochofengichtgases und/oder eine Teilmenge des im Konverterstahlwerk anfallenden Konvertergases mit einer Teilmenge des in der Koksofenanlage entstehenden Koksofengases gemischt werden und das Mischgas als Nutzgas verwendet werden. Zur Erzeugung eines Synthesegases, beispielsweise für die Ammoniaksynthese, kann als Rohgas eine Mischung aus Koksofengas und Hochofengichtgas oder ein Mischgas aus Koksofengas, Konvertergas und Hochofengichtgas verwendet werden. Zur Herstellung von Kohlenwasserstoffverbindungen eignet sich ein Mischgas aus Koksofengas und Konvertergas oder ein Mischgas aus Koksofengas, Konvertergas und Hochofengichtgas.

Zum Betrieb der biotechnologischen Anlage wird vorzugsweise Konvertergas, Hochofengichtgas oder ein Mischgas aus diesen beiden Gaskomponenten eingesetzt. Das Koksofengas ist für den biotechnischen Prozess nicht oder weniger geeignet. Insofern kann es zweckmäßig sein, in der Chemieanlage und in der biotechnologischen Anlage Nutzgasströme einzusetzen, die sich hinsichtlich ihrer Zusammensetzung unterscheiden.

Die Rohgase - Koksofengas, Konvertergas und/oder Hochofengichtgas - können einzeln oder in Kombination als Mischgas aufbereitet und dann als Synthesegas in der Chemieanlage und der biotechnologischen Anlage eingesetzt werden. Die Aufbereitung insbesondere von Koksofengas umfasst eine Gasreinigung zur Abtrennung störender Inhaltsstoffe, insbesondere Teer, Schwefel und Schwefelverbindungen, aromatischen Kohlenwasserstoffen (BTX) und hochsiedenden Kohlenwasserstoffen. Zur Herstellung des Synthesegases ist ferner eine Gaskonditionierung notwendig. Im Rahmen der Gaskonditionierung wird der Anteil der Komponenten CO, CO₂, H₂ innerhalb des Rohgases verändert. Die Gaskonditionierung umfasst beispielsweise eine Druckwechseladsorption zur Abtrennung und Anreichung von H₂ und/oder eine Wasser-Gas-Shift-Reaktion zur Umwandlung von CO in Wasserstoff und/oder einen Steam-Reformer zur Umwandlung des CH₄-Anteils in CO und Wasserstoff im Koksofengas..

Der in der Chemieanlage verwendete erste Teilstrom des Nutzgases kann mit Wasserstoff angereichert werden, welcher in einer zugeschalteten Anlage erzeugt wird. Die Wasserstofferzeugung erfolgt vorzugsweise durch Wasserelektrolyse, wobei die Wasserelektrolyse mit elektrischem Strom aus regenerativen Quellen betrieben werden kann. Bei der Wasserelektrolyse entsteht auch Sauerstoff, der im Hochofen zur Roheisenerzeugung und/oder im Konverterstahlwerk zur Rohstahlerzeugung genutzt werden kann.

Unter die Erfindung fällt ferner die Verwendung einer Chemieanlage im Verbund mit einer biotechnologischen Anlage zur Ankopplung an ein Hüttenwerk nach Anspruch 14.

Im Folgenden wird die Erfindung anhand einer lediglich ein Ausführungsbeispiel darstellenden Zeichnung erläutert. Es zeigen schematisch
- Fig. 1: ein stark vereinfachtes Blockschaltbild eines Anlagenverbundes zur Stahlerzeugung mit einem Hochofen zur Roheisenerzeugung und einem Konverterstahlwerk zur Rohstahlerzeugung, einem Kraftwerk, einer Chemieanlage und einer Biotechnologieanlage,
- Fig. 2: das stark vereinfachte Blockschaltbild eines Anlagenverbundes, der zusätzlich zu einem Hochofen zur Roheisenerzeugung, einem Konverterstahlwerk zur Rohstahlerzeugung, einem Kraftwerk, einer Chemieanlage und einer Biotechnologieanlage auch eine Koksofenanlage umfasst,

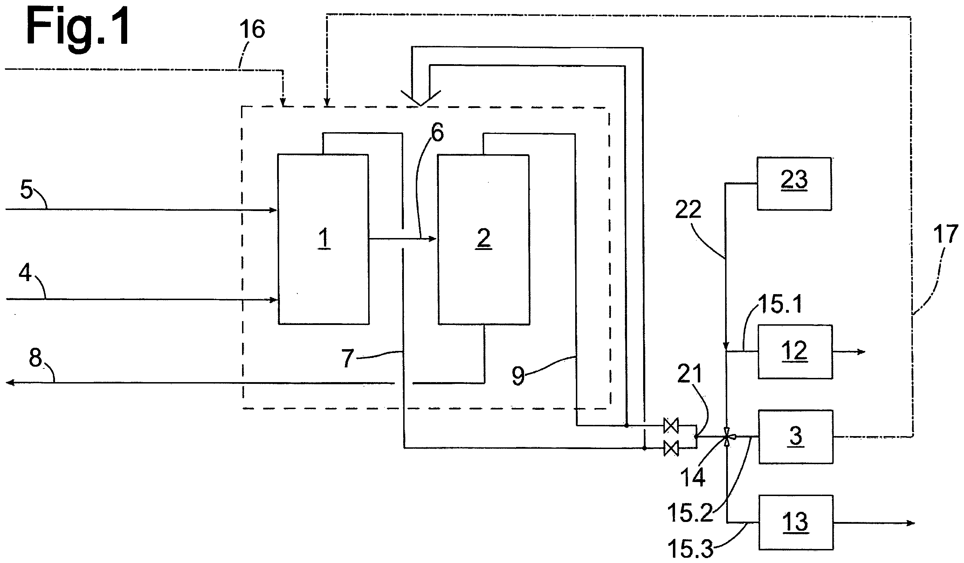

Der in Fig. 1 dargestellte Anlagenverbund zur Stahlerzeugung umfasst einen Hochofen 1 zur Roheisenerzeugung, ein Konverterstahlwerk 2 zur Rohstahlerzeugung und ein Kraftwerk 3 zur Stromerzeugung.

Im Hochofen 1 wird im Wesentlichen aus Eisenerz 4 und Reduktionsmitteln 5, insbesondere Koks und Kohle, Roheisen 6 gewonnen. Durch Reduktionsreaktionen entsteht ein Hochofengichtgas 7, welches als Hauptbestandteile Stickstoff, CO, CO₂ und einen geringen Anteil H₂ enthält. Im Konverterstahlwerk 2, das dem Hochofenprozess nachgeschaltet ist, wird Roheisen 6 zu Rohstahl 8 umgewandelt. Durch Aufblasen von Sauerstoff auf das flüssige Roheisen werden störende Verunreinigungen, insbesondere Kohlenstoff, Silizium und Phosphor entfernt. Zur Kühlung kann Schrott in Mengen bis zu 25 % bezogen auf die Roheisenmenge zugeführt werden. Ferner werden Kalk zur Schlackenbildung und Legierungsmittel zugegeben. Am Kopf des Konverters wird ein Konvertergas 9 abgezogen, welches einen sehr hohen Anteil an CO aufweist.

Das Kraftwerk 3 ist als Gasturbinenkraftwerk oder Gasturbinen- und Dampfturbinenkraftwerk ausgelegt und wird mit einem Gas betrieben, welches zumindest eine Teilmenge des bei der Roheisenerzeugung im Hochofen 1 anfallenden Hochofengichtgases 7 und eine Teilmenge des im Konverterstahlwerk 2 anfallenden Konvertergases 9 umfasst. Zur Führung der Gase ist ein Gasleitungssystem vorgesehen.

Gemäß einer in Fig. 1 dargestellten Gesamtbilanz wird dem Anlagenverbund Kohlenstoff als Reduktionsmittel 5 in Form von Kohle und Koks sowie Eisenerz 4 zugeführt. Als Produkte fallen Rohstahl 8 und Rohgase 7 und 9 an, die sich in Menge, Zusammensetzung, Heizwert und Reinheit unterscheiden und an verschiedenen Stellen im Anlagenverbund wieder eingesetzt werden. Bei einer Gesamtbetrachtung wird 40 bis 50 %, zumeist etwa 45 %, der Rohgase 7 und 9 wieder in den Hüttenprozess zur Roheisenerzeugung oder Rohstahlerzeugung zurückgeführt. Zwischen 50 und 60 %, zumeist etwa 55 %, der Rohgase 7 und 9 kann zum Betrieb des Kraftwerkes 3 genutzt werden.

Gemäß der Darstellung in Fig. 1 umfasst der Anlagenverbund zusätzlich eine Chemieanlage 12 und eine biotechnologische Anlage 13, wobei das Kraftwerk 3, die Chemieanlage 12 und die biotechnologische Anlage 13 hinsichtlich der Gasversorgung in einer Parallelschaltung angeordnet sind. Das Gasleitungssystem weist eine betrieblich steuerbare Gasverteilungsvorrichtung 14 zur Aufteilung der dem Kraftwerk 3, der Chemieanlage 12 und der biotechnologischen Anlage 13 zugeführten Gasmengenströme auf. In Strömungsrichtung vor der Gasverteilungsvorrichtung 14 kann eine Mischvorrichtung 21 zur Herstellung eines aus Hochofengichtgas 7, Konvertergas 9 bestehenden Mischgases vorhanden sein.

Das Hochofengichtgas 7 und das Konvertergas 9 können beliebig miteinander kombiniert werden. Die Kombination der Gasströme 7, 9 richtet sich nach dem gewünschten Synthesegas bzw. dem Produkt, welches in der Chemieanlage 12 hergestellt werden soll. Im Rahmen der Erfindung liegt es auch, dass der biotechnologischen Anlage 13 ein Gasstrom zugeführt wird, dessen Zusammensetzung sich von der in der Chemieanlage 12 eingesetzten Gaszusammenfassung unterscheidet.

Bei dem in Fig. 1 dargestellten Anlagenverbund wird zumindest eine Teilmenge des bei der Roheisenerzeugung im Hochofen 3 anfallenden Hochofengichtgases 7 und/oder eine Teilmenge des bei der Rohstahlerzeugung anfallenden Konvertergases 9 zum Betrieb des Kraftwerkes 3, der Chemieanlage 12 und der biotechnologischen Anlage 13 verwendet. Ein erster Teilstrom 15.1 des Nutzgases wird der Chemieanlage 12 zugeführt und nach einer Gasaufbereitung als Synthesegas zur Herstellung chemischer Produkte verwendet. Ein zweiter Teilstrom 15.2 des Nutzgases wird im Kraftwerk 3 zur Stromerzeugung eingesetzt. Ein dritter Teilstrom 15.3 des Nutzgases wird der biotechnologischen Anlage 13 zugeführt und für biochemische Prozesse verwendet.

Zur Deckung des Strombedarfes des Anlageverbundes wird extern bezogener Strom 16 und Kraftwerkstrom 17, der von dem Kraftwerk 3 des Anlagenverbundes erzeugt wird, herangezogen. Der extern bezogene Strom 16 wird vorzugsweise vollständig oder zumindest teilweise aus erneuerbarer Energie gewonnen und stammt beispielsweise aus Windkraftanlagen, Solaranlagen, Wasserkraftwerken und dergleichen. Zur Erreichung eines möglichst wirtschaftlichen Betriebs des Anlagenverbundes wird Strom in Zeiten niedriger Strompreise als externer Strom 16 zugekauft und der Kraftwerksprozess zur Stromversorgung gedrosselt. In Zeiten hoher Strompreise wird der im Kraftwerk 3 zur Stromerzeugung eingesetzte Teilstrom 15.2 des Nutzgases vergrößert.

Im Falle einer Änderung des dem Kraftwerk 3 zugeführten Gasstromes werden der zweite Teilstrom 15.2 und der dritte Teilstrom 15.3 des Nutzgases wechselweise verändert, so dass die Chemieanlage 12 mit einer Teilstrommenge 15.1 des Nutzgases betrieben werden kann, die geringeren betrieblichen Schwankungen ausgesetzt ist als der in der biotechnologischen Anlage 13 verwendete Nutzgasteilstrom 15.3. Der dritte Nutzgasteilstrom 15.3 wird zweckmäßig so geregelt, dass der in der Chemieanlage 12 eingesetzte erste Nutzgasteilstrom 15.1 mit einer Schwankungsbreite von ± 20 % konstant bleibt.

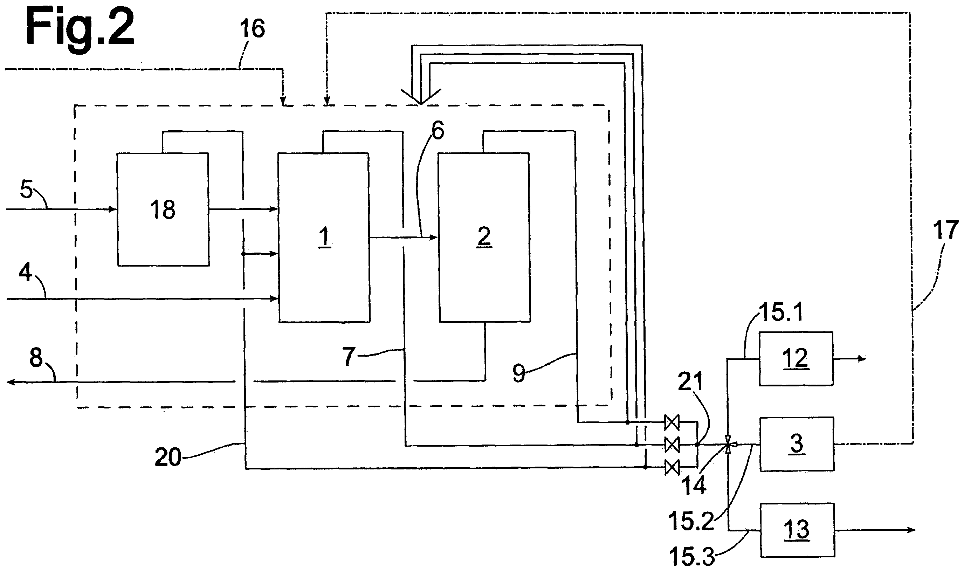

Im Ausführungsbeispiel der Fig. 2 umfasst der Anlagenverbund zusätzlich eine Koksofenanlage 18. Bei der Verkokung von Kohle zu Koks fällt Koksofengas 20 an, welches einen hohen Anteil an Wasserstoff und CH₄ enthält. Teile des Koksofengases 20 können für die Beheizung der Winderhitzer im Hochofen 1 genutzt werden. Das Gasleitungsystem schließt eine Gasverteilung für das Koksofengas 20 ein. In Strömungsrichtung vor der Gasverteilungsvorrichtung 14 kann eine Mischvorrichtung 21 zur Herstellung eines aus Hochofengichtgas 7, Konvertergas 9 und Koksofengas 20 bestehenden Mischgases vorhanden sein.

Das Hochofengichtgas 7, das Konvertergas 9 und das Koksofengas 20 können beliebig miteinander kombiniert werden. Die Kombination der Gasströme 7, 9, 20 richtet sich nach dem gewünschten Synthesegas bzw. dem Produkt, welches in der Chemieanlage 12 hergestellt werden soll. Im Rahmen der Erfindung liegt es auch, dass der biotechnologischen Anlage 13 ein Gasstrom zugeführt wird, dessen Zusammensetzung sich von der in der Chemieanlage 12 eingesetzten Gaszusammenfassung unterscheidet.

Auch bei dem in Fig. 2 dargestellten Anlagenkonzept wird ein erster Teilstrom 15.1 des Nutzgases der Chemielanlage 12 zugeführt und nach einer Gasaufbereitung als Synthesegas zur Herstellung chemischer Produkte verwendet. Ein zweiter Teilstrom 15.2 des Nutzgases wird im Kraftwerk 3 zur Stromerzeugung eingesetzt. Ein dritter Teilstrom 15.3 des Nutzgases wird der biotechnologischen Anlage 13 zugeführt und für biochemische Prozesse verwendet. Im Falle einer Änderung des dem Kraftwerk 3 zugeführten Gasstromes werden der zweite Teilstrom 15.2 und der dritte Teilstrom 15.3 des Nutzgases wechselweise verändert, so dass die Chemieanlage 12 mit einer Teilstrommenge 15.1 des Nutzgases betrieben werden kann, die geringeren betrieblichen Schwankungen ausgesetzt ist als der in der biotechnologischen Anlage verwertete Nutzgasteilstrom 15.3.

Der in der Chemieanlage 12 verwendete erste Teilstrom 15.1 des Nutzgases kann ferner mit Wasserstoff 22 angereichert werden, welcher in einer optional vorgesehenen zugeschalteten Anlage zur Wasserstofferzeugung 23 erzeugt wird.

## Patentansprüche

1. Anlagenverbund zur Stahlerzeugung mit
einem Hochofen (1) zur Roheisenerzeugung,
einem Konverterstahlwerk (2) zur Rohstahlerzeugung,
einem Gasleitungssystem für Gase, die bei der Roheisenerzeugung und/oder der Rohstahlerzeugung anfallen, und
einem Kraftwerk (3) zur Stromerzeugung,
wobei das Kraftwerk (3) als Gasturbinenkraftwerk oder Gasturbinen- und Dampfturbinenkraftwerk ausgelegt ist und mit einem Gas betrieben wird, welches zumindest eine Teilmenge des bei der Roheisenerzeugung im Hochofen anfallenden Hochofengichtgases (7) und/oder eine Teilmenge des in dem Konverterstahlwerk (2) anfallenden Konvertergases (9) umfasst, **dadurch gekennzeichnet, dass** an das Gasleitungssystem eine Chemieanlage (12) und eine biotechnologische Anlage (13) angeschlossen ist, wobei das Kraftwerk (3), die Chemieanlage (12) und die biotechnologische Anlage (13) hinsichtlich der Gasversorgung in einer Parallelschaltung angeordnet sind, und dass das Gasleitungssystem eine betrieblich steuerbare Gasverteilungsvorrichtung (14) zur Aufteilung der dem Kraftwerk (3), der Chemieanlage (12) und der biotechnologischen Anlage (13) zugeführten Gasmengenströme umfasst.

2. Anlagenverbund nach Anspruch 1, **dadurch gekennzeichnet, dass** der Anlagenverbund eine Koksofenanlage (18) umfasst und dass das Gasleitungssystem eine Gasverteilung für Koksofengas (20), welches bei einem Verkokungsprozess in der Koksofenanlage (18) anfällt, einschließt.

3. Anlagenverbund nach Anspruch 1 oder 2, **dadurch gekennzeichnet, dass** das Gasleitungssystem in Strömungsrichtung vor der Gasverteilungsvorrichtung (14) eine Mischvorrichtung (21) zur Herstellung eines aus Hochofengichtgas (7) und/oder Konvertergas (9) und/oder Koksofengas (20) bestehenden Mischgases aufweist.

4. Anlagenverbund nach einem der Ansprüche 1 bis 3, **dadurch gekennzeichnet, dass** der Anlagenverbund zusätzlich eine Anlage (23) zur Wasserstofferzeugung aufweist, die durch eine wasserstoffführende Leitung (22) mit dem Gasleitungssystem verbunden ist.

5. Verfahren zum Betreiben eines Anlagenverbundes zur Stahlerzeugung, der zumindest einen Hochofen (1) zur Roheisenerzeugung, ein Konverterstahlwerk (2), ein Kraftwerk (3), eine Chemieanlage (12) und eine biotechnologische Anlage (13) aufweist,
a) wobei zumindest eine Teilmenge des bei der Roheisenerzeugung im Hochofen (1) anfallenden Hochofengichtgases (7) und/oder eine Teilmenge des bei der Rohstahlerzeugung anfallenden Konvertergases (9) als Nutzgas zum Betrieb des Kraftwerkes (3), der Chemieanlage (12) und der biotechnologischen Anlage (13) verwendet wird,
b) wobei ein erster Teilstrom (15.1) des Nutzgases der Chemieanlage (12) zugeführt wird und nach einer Gasaufbereitung als Synthesegas zur Herstellung chemischer Produkte verwendet wird,
c) wobei ein zweiter Teilstrom (15.2) des Nutzgases im Kraftwerk (3) zur Stromerzeugung eingesetzt wird,
d) wobei ein dritter Teilstrom (15.3) des Nutzgases der biotechnologischen Anlage (13) zugeführt und für biochemische Prozesse verwendet wird,
e) wobei im Falle einer Änderung des dem Kraftwerk (3) zugeführten Gasstromes der zweite Teilstrom (15.2) und der dritte Teilstrom (15.3) des Nutzgases wechselweise verändert werden, so dass die Chemieanlage (12) mit einer Teilstrommenge (15.1) des Nutzgases betrieben werden kann, die geringeren betrieblichen Schwankungen ausgesetzt ist als der in der biotechnologischen Anlage (13) verwertete Nutzgasteilstrom (15.3).

6. Verfahren nach Anspruch 5, **dadurch gekennzeichnet, dass** der dritte Nutzgasteilstrom so geregelt wird, dass der in der Chemieanlage (12) eingesetzte erste Nutzgasteilstrom (15.1) mit einer Schwankungsbreite von ± 20 % konstant bleibt.

7. Verfahren nach Anspruch 5 oder 6, **dadurch gekennzeichnet, dass** der Anlagenverbund zusätzlich eine Koksofenanlage (18) umfasst und dass eine Teilmenge des bei der Roheisenerzeugung anfallenden Hochofengichtgases (7) und/oder eine Teilmenge des im Konverterstahlwerk (2) anfallenden Konvertergases (9) mit einer Teilmenge des in der Koksofenanlage (18) entstehenden Koksofengases (20) gemischt wird und dass das Mischgas als Nutzgas verwendet wird.

8. Verfahren nach Anspruch 7, **dadurch gekennzeichnet, dass** Hochofengichtgas (7) und Konvertergas (9) gemischt werden, dass aus dem Mischgas nach einer Gaskonditionierung ein Synthesegas hergestellt wird und dass dem Synthesegas oder dem gereinigten Mischgas vor der Weiterverarbeitung zu dem Synthesegas zusätzlich aufbereitendes Koksofengas (20) zugemischt wird.

9. Verfahren nach Anspruch 7, **dadurch gekennzeichnet, dass** aus Hochofengichtgas (7) nach einer Gaskonditionierung ein Synthesegas hergestellt wird und dass dem Synthesegas oder dem gereinigten Hochofengichtgas (7) vor der Weiterverarbeitung zu dem Synthesegas zusätzlich aufbereitetes Koksofengas (20) zugemischt wird.

10. Verfahren nach Anspruch 7, **dadurch gekennzeichnet, dass** aus Konvertergas (9) nach einer Gaskonditionierung ein Synthesegas hergestellt wird und dass dem Synthesegas oder dem gereinigten Konvertergas (9) vor der Weiterverarbeitung zu dem Synthesegas zusätzlich aufbereitetes Koksofengas (20) zugemischt wird.

11. Verfahren nach einem der Ansprüche 5 bis 10, **dadurch gekennzeichnet, dass** der Strombedarf durch extern bezogenen Strom (16) und durch Kraftwerkstrom (17), der von dem Kraftwerk (3) des Anlagenverbundes erzeugt wird, gedeckt wird und dass die Leistung des Kraftwerkes (3) in Abhängigkeit des extern bezogenen Stromes (16) verändert sowie der dem Kraftwerk (3) zugeführte zweite Nutzgasstrom (15.2) entsprechend geregelt wird.

12. Verfahren nach Anspruch 11, **dadurch gekennzeichnet, dass** der extern bezogene Strom (16) vollständig oder zumindest teilweise aus erneuerbarer Energie gewonnen wird.

13. Verfahren nach einem der Ansprüche 5 bis 12, **dadurch gekennzeichnet, dass** der in der Chemieanlage (12) verwendete erste Teilstrom (15.1) des Nutzgases mit Wasserstoff angereichert wird.

14. Verwendung einer Chemieanlage im Verbund mit einer biotechnologischen Anlage zur Ankopplung an ein Hüttenwerk, welches zumindest einen Hochofen (1) zur Roheisenerzeugung, ein Konverterstahlwerk (2) und ein mit Gas betriebenes Kraftwerk (3) zur Stromerzeugung umfasst, mit der Maßgabe, dass eine Teilmenge zumindest des bei der Roheisenerzeugung anfallenden Hochofengichtgases (7) und/oder eine Teilmenge des bei der Rohstahlerzeugung anfallenden Konvertergases (9) als Nutzgas zum Betrieb des Kraftwerkes (3), der Chemieanlage (12) und der biotechnologischen Anlage (13) verwendet wird, dass die Chemieanlage (12), die biotechnologische Anlage (13) und das Kraftwerk (3) in Bezug auf die Gasführung parallel geschaltet sind und dass zumindest die der biotechnologischen Anlage (13) und dem Kraftwerk (3) zugeführten Teilströme (15.3, 15.2) des Nutzgases separat regelbar sind.

## Claims

1. Plant complex for producing steel comprising
a blast furnace (1) for producing pig iron,
a converter steel works (2) for producing crude steel,
a gas-conducting system for gases that occur in the production of pig iron and/or the production of crude steel, and
a power-generating plant (3) for producing electricity,
the power-generating plant (3) being designed as a gas-turbine power-generating plant or gas-turbine and steam-turbine power-generating plant and being operated with a gas that comprises at least a partial amount of the blast-furnace top gas (7) that occurs in the production of pig iron in the blast furnace and/or a partial amount of the converter gas (9) that occurs in the converter steel works (2), **characterized in that** a chemical plant (12) and a biotechnological plant (13) are connected to the gas-conducting system, the power-generating plant (3), the chemical plant (12) and the biotechnological plant (13) being arranged in a parallel setup with respect to the gas supply, and **in that** the gas-conducting system comprises an operationally controllable gas-distributing device (14) for dividing the streams of gas that are fed to the power-generating plant (3), the chemical plant (12) and the biotechnological plant (13).

2. Plant complex according to Claim 1, **characterized in that** the plant complex comprises a coke-oven plant (18) and **in that** the gas-conducting system includes a gas distribution for coke-oven gas (20) that occurs in a coking process in the coke-oven plant (18).

3. Plant complex according to Claim 1 or 2, **characterized in that** the gas-conducting system has upstream of the gas-distributing device (14) in the direction of flow a mixing device (21) for producing a mixed gas consisting of blast-furnace top gas (7) and/or converter gas (9) and/or coke-oven gas (20).

4. Plant complex according to one of Claims 1 to 3, **characterized in that** the plant complex additionally has a plant (23) for producing hydrogen, which is connected to the gas-conducting system by a hydrogen-carrying line (22).

5. Method for operating a plant complex for steel production which has at least one blast furnace (1) for producing pig iron, a converter steel works (2), a power-generating plant (3), a chemical plant (12) and a biotechnological plant (13),
a) at least a partial amount of the blast-furnace top gas (7) that occurs in the production of pig iron in the blast furnace (1) and/or a partial amount of the converter gas (9) that occurs in the production of crude steel being used as a useful gas for operating the power-generating plant (3), the chemical plant (12) and the biotechnological plant (13),
b) a first partial stream (15.1) of the useful gas being fed to the chemical plant (12) and used after a gas-conditioning operation as syngas for producing chemical products,
c) a second partial stream (15.2) of the useful gas being used in the power-generating plant (3) for producing electricity,
d) a third partial stream (15.3) of the useful gas being fed to the biotechnological plant (13) and used for biochemical processes,
e) in the case of a change of the gas stream fed to the power-generating plant (3), the second partial stream (15.2) and the third partial stream (15.3) of the useful gas being changed alternately, so that the chemical plant (12) can be operated with a partial stream (15.1) of the useful gas that is subject to less operational fluctuations than the partial stream of useful gas (15.3) that is used in the biotechnological plant (13).

6. Method according to Claim 5, **characterized in that** the third partial stream of useful gas is controlled in such a way that the first partial stream of useful gas (15.1), used in the chemical plant (12), is operated constantly with a range of fluctuation of ±20%.

7. Method according to Claim 5 or 6, **characterized in that** the plant complex additionally comprises a coke-oven plant (18) and **in that** a partial amount of the blast-furnace top gas (7) that occurs in the production of pig iron and/or a partial amount of the converter gas (9) that occurs in the converter steel works (2) is mixed with a partial amount of the coke-oven gas (20) that occurs in the coke-oven plant (18) and **in that** the mixed gas is used as a useful gas.

8. Method according to Claim 7, **characterized in that** blast-furnace top gas (7) and converter gas (9) are mixed, **in that** a syngas is produced from the mixed gas after a gas-conditioning operation and **in that** conditioned coke-oven gas (20) is additionally admixed with the syngas or the cleaned mixed gas before the further processing to form the syngas.

9. Method according to Claim 7, **characterized in that** a syngas is produced from blast-furnace top gas (7) after a gas-conditioning operation and **in that** conditioned coke-oven gas (20) is additionally admixed with the syngas or the cleaned blast-furnace top gas (7) before the further processing to form the syngas.

10. Method according to Claim 7, **characterized in that** a syngas is produced from converter gas (9) after a gas-conditioning operation and **in that** conditioned coke-oven gas (20) is additionally admixed with the syngas or the cleaned converter gas (9) before the further processing to form the syngas.

11. Method according to one of Claims 5 to 10, **characterized in that** the demand for electricity is covered by externally obtained electricity (16) and by power-generating plant electricity (17), which is produced by the power-generating plant (3) of the plant complex, and **in that** the power output of the power-generating plant (3) is changed in dependence on the externally obtained electricity (16) and the second stream of useful gas (15.2), fed to the power-generating plant (3), is controlled correspondingly.

12. Method according to Claim 11, **characterized in that** the externally obtained electricity (16) is obtained completely or at least partially from renewable energy.

13. Method according to one of Claims 5 to 12, **characterized in that** the first partial stream (15.1) of the useful gas, used in the chemical plant (12), is enriched with hydrogen.

14. Use of a chemical plant in combination with a biotechnological plant for coupling to a metallurgical plant that comprises at least one blast furnace (1) for producing pig iron, a converter steel works (2) and a gas-operated power-generating plant (3) for producing electricity, with the proviso that a partial amount at least of the blast-furnace top gas (7) that occurs in the production of pig iron and/or a partial amount of the converter gas (9) that occurs in the production of crude steel is used as useful gas for operating the power-generating plant (3), the chemical plant (12) and the biotechnological plant (13), in that the chemical plant (12), the biotechnological plant (13) and the power-generating plant (3) are connected in parallel with respect to the carrying of gas and in that at least the partial streams (15.3, 15.2) of the useful gas that are fed to the biotechnological plant (13) and the power-generating plant (3) can be controlled separately.

## Revendications

1. Ensemble d'installations permettant la production d'acier, avec
un haut-fourneau (1) pour la production de fonte, une aciérie à convertisseurs (2) pour la production d'acier brut,
un réseau de conduites de gaz pour des gaz qui sont produits lors de la production de fonte et/ou lors de la production d'acier, et
une centrale électrique (3) pour la production d'électricité,
dans lequel la centrale électrique (3) est conçue comme une centrale électrique à turbines à gaz ou comme une centrale électrique à turbines à gaz et turbines à vapeur et elle fonctionne avec un gaz, qui comprend au moins une quantité partielle du gaz de gueulard de haut-fourneau (7) produit lors de la production de fonte dans le haut-fourneau et/ou une quantité partielle du gaz de convertisseur (9) produit dans l'aciérie à convertisseurs (2), **caractérisé en ce qu'**une installation chimique (12) et une installation biotechnologique (13) sont raccordées au réseau de conduites de gaz, dans lequel la centrale électrique (3), l'installation chimique (12) et l'installation biotechnologique (13) sont disposées selon un raccordement en parallèle en ce qui concerne l'alimentation en gaz, et **en ce que** le réseau de conduites de gaz comprend un dispositif de distribution de gaz (14) pouvant être commandé de façon opérationnelle pour la répartition des courants de quantités de gaz envoyés à l'aciérie (3), à l'installation chimique (12) et à l'installation biotechnologique (13).

2. Ensemble d'installations selon la revendication 1, **caractérisé en ce que** l'ensemble d'installations comprend une installation de fours à coke (18) et **en ce que** le réseau de conduites de gaz inclut une distribution de gaz du gaz de four à coke (20), qui est produit lors d'un processus de cokéfaction dans l'installation de fours à coke (18).

3. Ensemble d'installations selon la revendication 1 ou 2, **caractérisé en ce que** le réseau de conduites de gaz présente dans la direction d'écoulement avant le dispositif de distribution de gaz (14) un dispositif de mélange (21) pour la production d'un gaz mixte composé de gaz de gueulard de haut-fourneau (7) et/ou de gaz de convertisseur (9) et/ou de gaz de four à coke (20).

4. Ensemble d'installations selon l'une quelconque des revendications 1 à 3, **caractérisé en ce que** l'ensemble d'installations présente en outre une installation (23) pour la production d'hydrogène, qui est raccordée au réseau de conduites de gaz par une conduite de transport d'hydrogène (22).

5. Procédé de conduite d'un ensemble d'installations permettant la production d'acier, qui présente au moins un haut-fourneau (1) pour la production de fonte, une aciérie à convertisseurs (2), une centrale électrique (3), une installation chimique (12) et une installation biotechnologique (13),
a) dans lequel on utilise au moins une quantité partielle du gaz de gueulard de haut-fourneau (7) produit lors de la production de fonte dans le haut-fourneau (1) et/ou une quantité partielle du gaz de convertisseur (9) produit lors de la production d'acier brut comme gaz utile lors du fonctionnement de l'aciérie (3), de l'installation chimique (12) et de l'installation biotechnologique (13),
b) dans lequel on envoie un premier courant partiel (15.1) du gaz utile à l'installation chimique (12) et on l'utilise après une préparation du gaz comme gaz de synthèse pour la production de produits chimiques,
c) dans lequel on emploie un deuxième courant partiel (15.2) du gaz utile dans la centrale électrique (3) pour la production d'électricité,
d) dans lequel on envoie un troisième courant partiel (15.3) du gaz utile à l'installation biotechnologique (13) et on l'utilise pour des processus biochimiques,
e) dans lequel, en cas de changement du courant de gaz envoyé à la centrale électrique (3), on change alternativement le deuxième courant partiel (15.2) et le troisième courant partiel (15.3) du gaz utile, de telle manière que l'installation chimique (12) puisse fonctionner avec un débit de courant partiel (15.1) du gaz utile, qui est exposé à de plus faibles fluctuations opérationnelles que le courant partiel de gaz utile (15.3) valorisé dans l'installation biotechnologique (13).

6. Procédé selon la revendication 5, **caractérisé en ce que** le troisième courant partiel de gaz utile est régulé de telle manière que le premier courant partiel de gaz utile (15.1) employé dans l'installation chimique (12) reste constant avec une marge de fluctuation de ± 20 %.

7. Procédé selon la revendication 5 ou 6, **caractérisé en ce que** l'ensemble d'installations comprend en outre une installation de fours à coke (18) et **en ce que** l'on mélange une quantité partielle du gaz de gueulard de haut-fourneau (7) produit lors de la production de fonte et/ou une quantité partielle du gaz de convertisseur (9) produit dans l'aciérie à convertisseurs (2) avec une quantité partielle du gaz de four à coke (20) produit dans l'installation de fours à coke (18) et **en ce que** l'on utilise le gaz mixte comme gaz utile.

8. Procédé selon la revendication 7, **caractérisé en ce que** l'on mélange du gaz de gueulard de haut-fourneau (7) et du gaz de convertisseur (9), **en ce que** l'on fabrique après un conditionnement du gaz un gaz de synthèse à partir du gaz mixte et **en ce que** l'on ajoute en plus au gaz de synthèse ou au gaz mixte épuré avant son traitement en gaz de synthèse du gaz de four à coke de préparation (20).

9. Procédé selon la revendication 7, **caractérisé en ce que** l'on fabrique un gaz de synthèse à partir du gaz de gueulard de haut-fourneau (7) après un conditionnement du gaz et **en ce que** l'on ajoute en plus au gaz de synthèse ou au gaz de gueulard de haut-fourneau épuré (7), avant son traitement en gaz de synthèse, du gaz de four à coke de préparation (20).

10. Procédé selon la revendication 7, **caractérisé en ce que** l'on fabrique un gaz de synthèse à partir du gaz de convertisseur (9) après un conditionnement du gaz et **en ce que** l'on ajoute en plus au gaz de synthèse ou au gaz de convertisseur épuré (9) avant son traitement en gaz de synthèse, du gaz de four à coke de préparation (20) .

11. Procédé selon l'une quelconque des revendications 5 à 10, **caractérisé en ce que** l'on couvre les besoins en électricité par du courant prélevé à l'extérieur (16) et par du courant de la centrale électrique (17) qui est produit par la centrale électrique (3) de l'ensemble d'installations, et **en ce que** l'on modifie la puissance de la centrale électrique (3) en fonction du courant prélevé à l'extérieur (16) et on régule en conséquence le deuxième courant de gaz utile (15.2) envoyé à la centrale électrique (3).

12. Procédé selon la revendication 11, **caractérisé en ce que** l'on produit le courant prélevé à l'extérieur (16) entièrement ou au moins partiellement à partir d'énergie renouvelable.

13. Procédé selon l'une quelconque des revendications 5 à 12, **caractérisé en ce que** l'on enrichit le premier courant partiel (15.1) du gaz utile utilisé dans l'installation chimique (12) avec de l'hydrogène.

14. Utilisation d'une installation chimique en association avec une installation biotechnologique en vue du couplage à une usine sidérurgique, qui comprend au moins un haut-fourneau (1) pour la production de fonte, une aciérie à convertisseurs (2) et une centrale électrique fonctionnant au gaz (3) pour la production d'électricité, avec la condition qu'une quantité partielle au moins du gaz de gueulard de haut-fourneau (7) produit lors de la fabrication de fonte et/ou une quantité partielle du gaz de convertisseur (9) produit lors de la fabrication d'acier brut soit utilisée comme gaz utile pour le fonctionnement de la centrale électrique (3), de l'installation chimique (12) et de l'installation biotechnologique (13), que l'installation chimique (12), l'installation biotechnologique (13) et la centrale électrique (3) soient raccordées en parallèle en ce qui concerne l'alimentation en gaz et qu'au moins les courants partiels (15.3, 15.2) du gaz utile envoyés à l'installation biotechnologique (13) et à la centrale électrique (3) puissent être régulés de façon séparée.
